(19) Europäisches Patentamt
European Patent Office
Office européen des brevets

(11) **EP 2 256 195 A1**

(12) **EUROPÄISCHE PATENTANMELDUNG**

(43) Veröffentlichungstag:
**01.12.2010 Patentblatt 2010/48**

(51) Int Cl.:
***C12N 15/10*** *(2006.01)*

(21) Anmeldenummer: **09007338.8**

(22) Anmeldetag: **03.06.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(30) Priorität: **12.05.2009 EP 09160078**

(71) Anmelder: **Qiagen GmbH**
**40724 Hilden (DE)**

(72) Erfinder:
• **Fabis, Roland, Dr.**
  **51375 Leverkusen (DE)**
• **Wedler, Holger, Dr.**
  **40724 Hilden (DE)**
• **Petzel, Jan, Dr.**
  **40724 Hilden (DE)**

(74) Vertreter: **Roth, Carla et al**
**König-Szynka-Tilmann-von Renesse**
**Patentanwälte Partnerschaft**
**Lohengrinstraße 11**
**40549 Düsseldorf (DE)**

(54) **Nukleinsäureaufreinigungsverfahren**

(57) Die vorliegende Erfindung betrifft ein Verfahren zur Aufreinigung einer definierten Menge von Nukleinsäuren aus einer nukleinsäurehaltigen Probe, das wenigstens die nachfolgenden Schritte aufweist:

a. Inkontaktbringen der nukleinsäurehaltigen Probe mit einer definierten Menge einer nukleinsäurebindenden Phase mit folgenden Merkmalen:

(i) die nukleinsäurebindende Phase weist nukleinsäurebindende Liganden auf, die wenigstens eine protonierbare Gruppe aufweisen;

(ii) die nukleinsäurebindenden Liganden liegen an einen Träger gebunden vor;

(iii) die nukleinsäurebindende Phase weist eine Oberfläche mit einer niedrigen Ladungsdichte auf,

wobei die Menge an Nukleinsäuren in der Probe die Bindungskapazität der eingesetzten Menge an nukleinsäurebindender Phase übersteigt;

b. Bindung der Nukleinsäuren an die nukleinsäurebindende Phase bei einem pH-Wert (Bindungs pH-Wert), der unterhalb des pKs-Wertes wenigstens einer der protonierbaren Gruppen liegt;

c. Elution der Nukleinsäuren bei einem pH-Wert, der oberhalb des Bindungs pH-Wertes liegt, wobei eine definierte Menge an Nukleinsäuren erhalten wird.

Darüber hinaus werden entsprechende Kits sowie nukleinsäurebindende Phasen, die zur Aufreinigung von Nukleinsäuren eingesetzt werden können, offenbart.

**Beschreibung**

Die vorliegende Erfindung betrifft ein Verfahren zur Aufreinigung einer definierten Menge von Nukleinsäuren aus einer nukleinsäurehaltigen Probe. Darüber hinaus werden geeignete Kits und nukleinsäurebindende Phasen zur Verfügung gestellt, die die Isolierung einer definierten Menge an Nukleinsäuren aus einer Probe erlauben und daher zur Normalisierung der Nukleinsäurekonzentration im Eluat geeignet sind.

Verschiedene Verfahren zur Aufreinigung und Isolierung von Nukleinsäuren sind im Stand der Technik bekannt. Diese beinhalten den Einsatz von Phenol-Chloroform, Aussalzverfahren, den Einsatz von Ionenaustauschern sowie Silikapartikel. Ein bekanntes Verfahren zur Nukleinsäureaufreinigung ist das sog. "Charge-Switch Verfahren". Danach wird eine nukleinsäurebindende Phase bei einem ersten pH-Wert mit einer nukleinsäurehaltigen Probe in Kontakt gebracht, bei der die nukleinsäurebindende Phase eine positive Ladung aufweist. Dies begünstigt die Bindung der negativ geladenen Nukleinsäuren an die Phase. Zur Freisetzung/Elution der Nukleinsäuren wird gemäß dem Charge-Switch Prinzip ein zweiter pH-Wert eingestellt, der höher als der pKs-Wert der nukleinsäurebindende Phase ist, um die positive Ladung zu invertieren, bzw. zu neutralisieren. Dies fördert die Ablösung der gebundenen Nukleinsäuren von der nukleinsäurebindenden Phase.

Für viele Analysetechniken und biologischen Verfahren muss eine bestimmte, d. h. definierte Menge an Nukleinsäure eingesetzt werden. Die Menge der isolierten oder amplifizierten Nukleinsäure aus unterschiedlichen Ausgangsmaterialien (Proben) hängt von einer Reihe schwer zu kontrollierender Faktoren ab, so dass die Ausbeuten je nach Methode und Material stark schwanken können. Um nachfolgende Experimente mit den isolierten Nukleinsäuren unter vergleichbaren Bedingungen durchführen zu können, ist in der Regel eine Quantifizierung der gewonnenen Nukleinsäure und anschließend ein Einstellen der Konzentrationen bzw. Nukleinsäuremenge auf einen bestimmten Wert und damit eine Normalisierung erforderlich. Für viele standardisierte Prozesse und insbesondere die Automatisierung von Prozessen ist eine solche Normalisierung oftmals zwingend notwendig, um reproduzierbare Ergebnisse zu erhalten.

Aus diesem Grunde gibt es eine Vielzahl von Quantifizierungsmethoden, um Nukleinsäuren in einer aufgereinigten Probe zu quantifizieren. Ein Beispiel für eine übliche Quantifizierungsmethode ist die spektrophotometrische Bestimmung der DNA Menge in einer Probe. Im Anschluss an die Bestimmung der Konzentration der Nukleinsäure in der Probe kann die Konzentration dann einheitlich angepasst werden. Andere bekannte Nukleinsäurequantifizierungsmethoden basieren auf interkalierenden Farbstoffen wie bspw. Ethidiumbromid, SYBR Green oder Picogreen. Durch Vergleich einer Standardkurve mit den gemessenen Werten kann die Konzentration bestimmt werden.

Neben den beschriebenen spektrometrischen oder auf Fluoreszenz basierenden Quantifizierungsmethoden sind im Stand der Technik auch verschiedene Aufreinigungsverfahren bekannt, die eine Präparation von einheitlichen Konzentrationen an Nukleinsäuren aus unterschiedlichen Ausgangsmaterialien erlauben sollen. Beispiele sind das MagneSil-Kit von Promega oder das SequalPrep-Kit von Invitrogen.

Das MagneSil-Kit von Promega wird bspw. verwendet, um genomische DNA aus Vollblut zu isolieren. Das Kit basiert auf der Bindung von DNA an Silika beschichtete paramagnetische Partikel unter chaotropen Salzbedingungen. Als Ergebnis erhält man üblicher Weise 1 μg (+/- 50 %) gereinigte genomische DNA nach Elution mit einem Niedrigsalzpuffer oder Wasser. Aufgrund der recht großen Schwankungen von bis zu 50 % ist diese Methode jedoch verbesserungsbedürftig.

Das SequalPrep-Kit von Invitrogen basiert auf der Bindung von DNA an Oberflächen, die mit einem Ionenaustauscher beschichtet sind. Die DNA bindet bei einem sauren pH-Wert und die Elution erfolgt mit einem stark alkalischen Puffer, dessen pH-Wert über dem pks-Wert des Ionenaustauschers liegt. Dieses Kit basiert auf der sogenannten Charge-Switch Technologie (siehe oben). Anwendung findet das Kit bei der Aufreinigung und Normalisierung von long range (LR) PCRs und es ist auch für die Template Vorbereitung für Sequenzierungen geeignet. Der Hersteller gibt die Ausbeuten von ca. 25 ng mit einer zwei bis dreifachen Schwankung bei mindestens 250 ng Ausgangs-DNA an. Das bedeutet, dass auch hier die Schwankungen recht hoch sein können, so dass auch hier Optimierungsbedarf besteht.

Die bekannten Aufreinigungsverfahren weisen daher in der Praxis immer noch starke Schwankungen in den Ausbeuten auf. Schwierigkeiten treten insbesondere dann auf, wenn unterschiedliche Mengen an Ausgangsmaterial oder unterschiedliche Ausgangsmaterialien zum Einsatz kommen.

Neben dem Erhalt einer aufgereinigten Nukleinsäure ist es daher wünschenswert, bei der Isolierung auch eine definierte Menge an Nukleinsäuren aufzureinigen und damit für ein Probenmaterial bereits ein Eluat zu erhalten, das zwischen verschiedenen Proben eine annähernd gleiche Konzentration an Nukleinsäuren mit möglichst geringen Konzentrationsschwankungen aufweist.

Darüber hinaus ist es wünschenswert, dass die aufgereinigten Nukleinsäuren unmittelbar für die weiteren Anwendungen einsatzfertig sind, d.h. bspw. keine weiteren Umpufferungen oder ähnliches erforderlich sind. Dies ist basierend auf den im Stand der Technik bekannten Aufreinigungsverfahren nicht immer möglich, da diese zur Elution der gereinigten Nukleinsäuren oft hohe pH-Werte und/oder hohe Salzkonzentrationen erforderlich machen. Daher ist es oftmals notwendig, die aufgereinigte Nukleinsäure zuvor zu fällen oder aber beispielsweise den pH-Wert für die nachfolgenden Anwendungen (downstream Applikationen) einzustellen, wodurch sich jedoch auch wieder die Konzentration im Eluat verändern kann.

Auch wenn die bekannten Verfahren zur Aufreinigung von Nukleinsäuren und mit gewissen Einschränkungen auch zur Aufreinigung einer definierten Menge von Nukleinsäuren geeignet sind, besteht das Bedürfnis, die bestehenden Verfahren zu verbessern, insbesondere die Aufreinigung einer definierten Menge an Nukleinsäuren aus verschiedenen Proben zu ermöglichen, bei der nur geringe Schwankungen in der Konzentration der aufgereinigten Nukleinsäure auftreten.

Der vorliegenden Erfindung liegt daher die Aufgabe zugrunde, die bestehenden Verfahren zur entsprechenden Aufreinigung von Nukleinsäuren zu verbessern.

Die vorliegende Erfindung löst diese Aufgabe mit einem Verfahren zur Isolierung und/oder Aufreinigung einer definierten Menge von Nukleinsäuren aus einer nukleinsäurehaltigen Probe, das wenigstens die nachfolgenden Schritte aufweist:

a. Inkontaktbringen der nukleinsäurehaltigen Probe mit einer definierten Menge einer nukleinsäurebindenden Phase mit folgenden Merkmalen:

(i) die nukleinsäurebindende Phase weist nukleinsäurebindende Liganden auf, die wenigstens eine protonierbare Gruppe aufweisen;
(ii) die nukleinsäurebindenden Liganden liegen an einen Träger gebunden vor,
(iii) die nukleinsäurebindende Phase weist eine Oberfläche mit einer niedrigen Ladungsdichte auf,

wobei die Menge an Nukleinsäuren in der Probe die Bindungskapazität der eingesetzten nukleinsäurebindender Phase übersteigt;
b. Bindung der Nukleinsäuren an die nukleinsäurebindende Phase bei einem pH-Wert (Bindungs pH-Wert), der unterhalb des pKs-Wertes wenigstens einer der protonierbaren Gruppen liegt;
c. Elution der Nukleinsäuren bei einem pH-Wert, der oberhalb des Bindungs pH-Wertes liegt, wobei eine definierte Menge an Nukleinsäuren erhalten wird.

Die vorliegende Erfindung betrifft die Isolierung und/oder Aufreinigung von Nukleinsäuren mittels einer speziellen nukleinsäurebindenden Phase, welche die Aufreinigung/Isolierung einer definierten Menge an Nukleinsäuren aus einer Probe mit geringen Konzentrationsschwankungen erlaubt. Entscheidend hierfür ist die Ausgestaltung der nukleinsäurebindenden Phase. Zur Bindung der Nukleinsäuren aus der Probe weist diese nukleinsäurebindende Liganden auf, die wenigstens eine protonierbare Gruppe aufweisen. Geeignete protonierbare Gruppen, insbesondere Aminogruppen, sind nachfolgend im Detail beschrieben. Die Bindung der Nukleinsäuren erfolgt bei einem pH-Wert unterhalb des pKs-Wertes wenigstens einer dieser protonierbaren Gruppen. Die protonierbaren Gruppen nehmen ein oder mehrere Protonen auf und werden dadurch positiv geladen, was dazu führt, dass die nukleinsäurebindende Phase die negativ geladenen Nukleinsäuren binden kann. Die Elution erfolgt bei einem höheren pH-Wert, wodurch die positive Ladung der protonierbaren Gruppe geringer und die an die nukleinsäurebindende Phase gebundene Nukleinsäure freigesetzt wird. Gemäß einigen Ausführungsformen kann die protonierbare Gruppe bei der Elution sogar neutral oder ggf. auch negativ geladen sein. Das Verfahren kann zur Isolierung und/oder Aufreinigung von Nukleinsäuren aus einer biologischen Probe eingesetzt werden. Darüber hinaus kann das Verfahren im Anschluss an ein klassisches Nukleinsäureaufreinigungsverfahren durchgeführt werden, um bspw. bereits aufgereinigte Nukleinsäuren zu normalisieren und so eine definierte Menge an Nukleinsäuren zu erhalten.

Ein wesentliches Merkmal der vorliegenden Erfindung ist die Oberfläche der nukleinsäurebindenden Phase. Diese weist erfindungsgemäß eine niedrige Ladungsdichte auf. Diese spezielle Oberfläche hat in Kombination mit dem Merkmal, dass die Menge an Nukleinsäuren in der Probe die Bindungskapazität der eingesetzten Menge an nukleinsäurebindender Phase übersteigt, überraschender Weise den Effekt, dass eine definierte Menge an Nukleinsäuren aus der Probe isoliert werden kann, wobei im Vergleich mit dem Stand der Technik deutlich geringere Schwankungen in der Nukleinsäurekonzentration auftreten.

Im Unterschied zum Stand der Technik wird erfindungsgemäß nicht versucht, eine Oberfläche zu erzielen, welche die Bindung einer besonders großen Menge an Nukleinsäuren erlaubt. Vielmehr wird bewusst die Ladungsdichte auf der Oberfläche und damit die Nukleinsäurebindungskapazität der nukleinsäurebindenden Phase verringert. Daher kann nur eine begrenzte, aber für eine bestimmte Probe immer gleiche d.h. definierte Menge an Nukleinsäuren gebunden und entsprechend eluiert werden. Für ein bestimmtes biologisches Probenmaterial muss daher nur in vorteilhafter Weise einmalig die Bindekapazität bestimmt werden. Weitere Quantifizierungsschritte können entfallen, da eine bestimmte Menge an nukleinsäurebindender Phase nur eine definierte und daher immer gleiche Nukleinsäuremenge binden kann. Dadurch können in vorteilhafter Weise Arbeitsschritte - wie bspw. das Aufkonzentrieren oder das Verdünnen der aufgereinigten Proben entfallen, die andernfalls notwendig wären, um alle Proben auf die gleiche Konzentration an gereinigten Nukleinsäuren zu bringen.

Der Begriff "eine definierte Menge" bezieht sich insbesondere auf eine Konzentration an Nukleinsäuren, die innerhalb eines definierten, d.h. engen Schwankungsbereichs liegt. Wenn dieser für eine bestimmte Probe bzw. Probenmaterial nicht bekannt ist, kann dieser Bereich (experimentell) bestimmt werden. Vorzugsweise liegen die mit dem erfindungsgemäßen Verfahren erzielten Nukleinsäuremengen bzw. Konzentrationen in einem engen Bereich, so dass die Schwankungen je nach Probenmaterial bei weniger als +/- 30 %, vorzugsweise weniger als +/- 20 % und insbesondere in einem Bereich von +/- 15 % und besonders bevorzugt +/- 10 % liegen.

Die Menge der nukleinsäurebindenden Phase im Verhältnis zu der Menge/Konzentration der Nukleinsäuren im Probenmaterial sollte vorzugsweise so gewählt sein, dass man immer im Plateau bereich und somit im Sättigungsbereich der Nukleinsäurebindekapazität der nukleinsäurebindenden Phase liegt. Daher wird die Menge an nukleinsäurebindender Phase (bspw. die Menge an Partikeln) so gewählt, dass die Nukleinsäuren in der Probe im Überschuss zu der Nukleinsäurebindekapazität der eingesetzten Menge an nukleinsäurebindender Phase vorliegen. Aufgrund der geringen Nukleinsäurebindekapazität der erfindungsgemäßen Partikel hat das erfindungsgemäße Verfahren deutliche Vorteile gegenüber dem Einsatz von nukleinsäurebindenden Phasen/Partikeln, die eine hohe Nukleinsäurebindekapazität aufweisen (bspw. Silikapartikel oder auf herkömmliche Weise mit Polyaminen beschichtete Partikel). Im Stand der Technik muss man entweder hohe Schwankungen in Kauf nehmen oder derart geringe Mengen an nukleinsäurebindender Phase/Partikel einsetzen, dass die Handhabung insbesondere im Rahmen eines automatisierten Prozesses erschwert wird. So müsste bspw. bei Einsatz der im Stand der Technik bekannten Partikel die eingesetzte Menge derart herunterverdünnt werden, dass sie nur schwierig in einem automatisierten Verfahren zu handhaben wären. Dies birgt wiederum Fehlerquellen, da der Verlust von bereits wenigen Partikeln mit hoher Nukleinsäurebindekapazität eine deutliche Schwankung in der Ausbeute an Nukleinsäuren zur Folge hätte. Auch unter diesen Gesichtspunkten ist das erfindungsgemäße Verfahren daher dem Stand der Technik überlegen.

Zur Aufreinigung einer definierten Menge an Nukleinsäuren und damit Normalisierung der Nukleinsäurekonzentration wird erfindungsgemäß der nukleinsäurebindenden Phase so viel Nukleinsäure angeboten, dass die Nukleinsäurebindekapazität der eingesetzten nukleinsäurebindenden Phase erschöpft ist. Der restliche, ungebundene Teil der Nukleinsäure wird bei der Aufreinigung verworfen. Optional kann die nukleinsäurebindende Phase mit der gebundenen Nukleinsäure vor der Elution gewaschen werden. Die Nukleinsäure wird im Elutionsschritt von der Oberfläche der nukleinsäurebindenden Phase in die Elutionslösung überführt. Dadurch wird für ein gegebenes Probenmaterial immer die gleiche, d.h. definierte Menge an Nukleinsäure bei der Aufreinigung erhalten.

Vorzugsweise wird die Oberfläche mir niedriger Ladungsdichte durch eines oder mehrere der nachfolgenden Merkmale erzielt:

a. die nukleinsäurebindenden Liganden weisen am Träger gebunden jeweils nicht mehr als ein oder zwei protonierbare Gruppen zur Bindung der Nukleinsäuren auf; und/oder

b. die nukleinsäurebindenden Liganden sind ausgewählt aus der Gruppe der Mono- und Diamine; und/oder

c. der Träger ist mit einer Mischung aus nukleinsäurebindenden Liganden und Verdünnungsliganden beschichtet; und/oder

d. die nukleinsäurebindenden Liganden liegen im Unterschuss an den Träger gebunden vor.

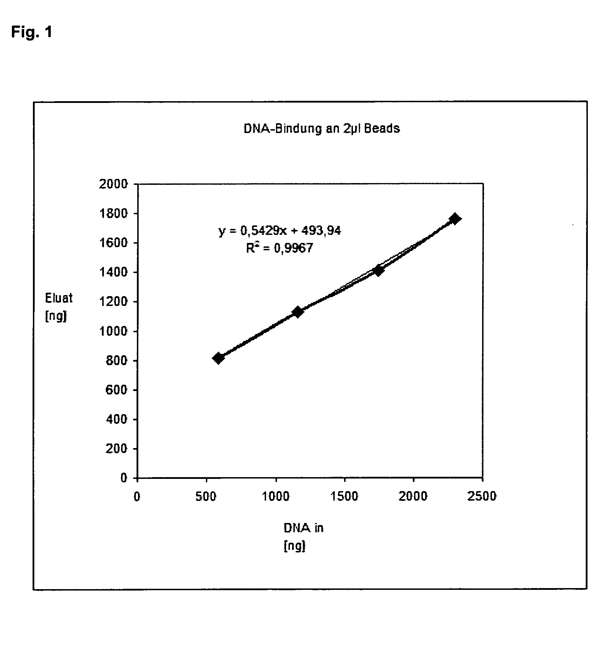

Erfindungsgemäß wird wie ausgeführt eine nukleinsäurebindende Phase eingesetzt, die eine Oberfläche mit niedriger Ladungsdichte aufweist. Hierfür weisen die nukleinsäurebindenden Liganden vorzugsweise jeweils nicht mehr als ein oder zwei protonierbare Gruppen wie bspw. Amino-Gruppen zur Bindung der Nukleinsäuren auf. Entsprechend sind die nukleinsäurebindenden Liganden vorzugsweise ausgewählt aus der Gruppe der Mono- und Diamine. Es hat sich gezeigt, dass im Gegensatz zu einer herkömmlichen Beschichtung mit Polyaminen wie Spermin und Spermidin Mono- bzw. Diamine sehr gut zur Normalisierung von Nukleinsäurekonzentrationen geeignet sind. Auf herkömmliche Weise mit Polyaminen beschichtete Träger weisen augenscheinlich eine zu große Ladungsdichte auf der Oberfläche auf, um für eine Normalisierung geeignet zu sein. Sie binden daher größere Mengen an Nukleinsäuren. Wird solch eine Oberfläche mit einer nukleinsäurehaltigen Probe in Kontakt gebracht, findet man eine starke Abhängigkeit der Bindekapazität von der Nukleinsäurekonzentration. Typischerweise zeigt sich im untersuchten Bereich ein proportionales Verhältnis zwischen angebotener und eluierter DNA-Menge. Fig. 1 zeigt am Beispiel von mit dem Polyamin Spermin beschichteten Polymerpartikeln dieses Verhalten, was die beschriebenen Schankungen in der Menge an aufgereinigter Nukleinsäure zur Folge hat und wodurch eine Normalisierung bei der Aufreinigung nicht möglich ist.

Durch die Verwendung von vorzugsweise Mono- oder Diaminen bei der Beschichtung des Trägers erzeugt man dagegen eine begrenzte, aber definierte Mengen an Ladungsträgern und damit nukleinsäurebindenden Gruppen auf der Oberfläche. Die so erzeugte Ladungsträgerdichte zur Bindung der Nukleinsäuren ist deutlich geringer als bei einer entsprechenden Beschichtung mit Polyaminen, wie z. B. Spermin oder gar Polyethyleniminen. Insbesondere mit N-Proply-1,3-Propandiaminen modifizierte Polymerpartikel sind gut geeignet, die Nukleinsäurekonzentration bereits bei der Aufreinigung zu normalisieren und damit eine definierte Menge an Nukleinsäuren aus einer Probe zu isolieren. Der Vorteil eines mit Monoaminen bzw. Diaminen beschichteten Trägers zur Herstellung einer nukleinsäurebindenden Phase liegt insbesondere darin, dass nur eine begrenzte, aber für eine bestimmte Probe innerhalb eines geringen Schwankungsbereichs gleiche Menge an Nukleinsäure gebunden wird. Durch die hohe Reproduzierbarkeit bei der Herstellung der Trägermaterialien und bei der Aminofunktionalisierung der Trägeroberfläche erreicht man eine enge Variabilität der Ladungsträgerdichte und damit der Nukleinsäurebindekapazität. Dadurch werden ungewünschte Schwankungen in der Nukleinsäurekonzentration bei der Aufreinigung vermieden. Die geschilderten Vorteile lassen sich nicht nur gegenüber Polyaminen aufzeigen, sondern zeigen sich insbesondere auch gegenüber den üblicherweise eingesetzten Silika-Partikeln, bei denen aufgrund der hohen Nukleinsäurebindekapazität der Oberfläche Schwankungen von bis zu 50 % bei der Isolierung der Nukleinsäuren aus gleichem Probenmaterial auftreten.

Überraschender Weise hat sich ferner gezeigt, dass eine niedrigere Ladungsdichte auf der Oberfläche des Trägers auch dadurch erreicht werden kann, dass sofern beispielsweise Polyamine als nukleinsäurebindende Liganden eingesetzt werden, der Träger nur mit einer geringen Menge an Polyaminen beschichtet wird, um die gewünschte geringe Ladungsdichte auf der Oberfläche des nukleinsäurebindenden Materials zu erhalten. Gemäß einer Ausführungsform werden die nukleinsäurebindenden Liganden daher beabstandet zueinander auf dem Trägermaterial angeordnet bzw. verdünnt. Um eine solche Anordnung der nukleinsäurebindenden Liganden zu erreichen, kann gemäß einer Ausführungsform der Träger nur mit geringen Mengen an nukleinsäurebindenden Liganden wie bspw. Polyaminen oder vorzugsweise Mono- und/oder Diaminen beschichtet werden. Vorzugsweise erfolgt die Funktionalisierung mit nukleinsäurebindenden Liganden daher im Unterschuss insbesondere in Relation zur Bindekapazität der Trägermaterialoberfläche. Dadurch wird quasi eine Verdünnung der nukleinsäurebindenden Liganden auf dem Träger erreicht, so dass weniger protonierbare Gruppen zur Verfügung stehen. Bspw. können nur $\leq 50\%$, $\leq 25\%$, $\leq 15\%$, $\leq 10\%$ oder nur $\leq 5\%$ der funktionellen bzw. mit den nukleinsäurebindenden Liganden funktionalisierbaren Gruppen auf dem Trägermaterial mit nukleinsäurebindenden Liganden funktionalisiert werden. Auch dadurch wird eine nukleinsäurebindende Phase mit einer geringen Ladungsdichte auf der Oberfläche erhalten.

Ferner kann die Beschichtung des Trägers mit einer Mischung aus nukleinsäurebindenden Liganden und sog. Verdünnungsliganden erfolgen. Der Begriff "Verdünnungsliganden" wird hier dazu verwendet, um die Funktion im Verhältnis zu den nukleinsäurebindenden Liganden darzustellen. Ihre Funktion ist es, die Menge an nukleinsäurebindenden Liganden auf dem Träger variieren und damit einstellen zu können und damit eine geringere Ladungsdichte auf der Oberfläche der nukleinsäurebindenden Phase einzustellen. Je höher der Anteil an Verdünnungsliganden im Verhältnis zu nukleinsäurebindenden Liganden ist, desto weniger nukleinsäurebindende Liganden werden auf den Träger aufgebracht und desto geringer ist die Nukleinsäurebindungskapazität. Die Verdünnungsliganden können negativ, positiv oder neutral geladen sein bzw. ionisierbare Gruppen aufweisen. Der Anteil an nukleinsäurebindenden Liganden im Verhältnis zu den Verdünnungsgruppen kann bspw. $\leq 50\%$, $\leq 25\%$, $\leq 15\%$, $\leq 10\%$ oder nur $\leq 5\%$ betragen. Geeignete Verdünnungsliganden sind bspw. Dimethylamin, Diethylamin und Ammoniak. Ein weiteres Beispiel für einen geeigneten Verdünnungsliganden wäre Ethanolamin. Gemäß einer bevorzugten Ausführungsform wird eine Mischung aus nukleinsäurebindenden Liganden und Verdünnungsliganden zur Beschichtung des Trägers eingesetzt.

Die Bindekapazität der nukleinsäurebindenden Phase liegt gemäß einer Ausführungsform in einem Bereich von 1 bis 500$\mu$g Nukleinsäure pro mg nukleinsäurebindender Phase, wobei geringere Bindekapazitäten wie 1 bis 300$\mu$g, vorzugsweise 1 bis 200$\mu$g, besonders bevorzugt 10 bis 100$\mu$g Nukleinsäure, insbesondere DNA, pro mg nukleinsäurebindender Phase besonders geeignet sind, um auch geringere Mengen an Nukleinsäuren zu normalisieren.

Um eine schonende Elution der Nukleinsäuren zu erreichen, erfolgt die Elution vorzugsweise bei einem pH-Wert, der oberhalb des Bindungs pH-Wertes liegt, jedoch wenigstens eine pH-Einheit, vorzugsweise wenigstens zwei pH-Einheiten unterhalb des pKs-Wertes wenigstens einer der protonierbaren Gruppen, vorzugsweise aller protonierbarer Gruppen liegt. Dies hat den erheblichen Vorteil, dass die Elution auch bei schonenden Bedingungen durchgeführt werden kann. Die vorliegende Erfindung erlaubt daher die Elution bei einem pH-Wert, der unterhalb der pKs-Werte der protonierbaren Gruppen liegt.

Gemäß einer Ausführungsform der vorliegenden Erfindung erfolgt die Bindung der Nukleinsäuren bei einem pH-Wert von 3 bis 8. Diese Angabe bezieht sich auf den pH-Wert während der Bindung und damit in der Probe. Das erfindungsgemäße Verfahren ist je nach Gestaltung der festen Phase auch bei sehr schonenden Bedingungen durchführbar, so dass die Bindung der Nukleinsäuren auch bei einem pH-Wert von 4 bis 7,5; vorzugsweise bei 5 bis 7,5; besonders bevorzugt bei 5 bis 7 und ganz besonders bevorzugt bei 6,5 bis 7 und damit im nahezu neutralen Bereich durchgeführt werden kann. Aufgrund der Tatsache, dass die protonierbaren Gruppen vorzugsweise einen pKs-Wert von 9 bis 12, weiter bevorzugt von 10 bis 12 aufweisen, liegen diese selbst bei relativ neutralen pH-Werten ausreichend positiv geladen vor, um die effektive Anbindung der Nukleinsäuren zu erlauben. Daher kann die Bindung unter sehr schonenden Bedingungen erfolgen, was eine Schädigung der Nukleinsäuren verhindert.

Darüber hinaus hat es sich als vorteilhaft erwiesen, die Bindung bei einer niedrigen Salzkonzentration vorzunehmen. Gemäß einer Ausführungsform liegt daher die Salzkonzentration bei der Bindung der Nukleinsäuren an die nukleinsäurebindende Phase bei $\leq 1$ M. Vorzugsweise liegt die Salzkonzentration bei $\leq 0,5$ M, $\leq 0,25$ M oder sogar $\leq 0,1$ M. Eine niedrige Salzkonzentration ist bevorzugt, um die Bindung der Nukleinsäuren an die feste Phase zu optimieren. Zu hohe Ionenkonzentrationen können die ionischen Wechselwirkungen von Nukleinsäure und der nukleinsäurebindenden Phase negativ beeinflussen. Es hat sich herausgestellt, dass der Bindungspuffer auch gewisse Mengen organische Substanzen wie bspw. Kohlenhydrate, Alkohole (wie bspw. Ethanol, Methanol), Aceton, Acetonitril oder Mischungen derselben enthalten kann. Diese beeinträchtigen die Bindung nicht.

Ein weiterer wesentlicher Schritt des vorliegenden Verfahrens ist die Elution der Nukleinsäuren. Wie dargelegt, erfolgt die Nukleinsäurefreisetzung bei einem pH-Wert, der oberhalb des Bindungs pH-Wertes liegt. Dies hat zur Folge, dass die protonierbaren Gruppen bei der Elution weniger positiv geladen vorliegen, wodurch die Freisetzung der Nukleinsäuren begünstigt wird. Darüber hinaus liegt der pH-Wert bei der Elution vorzugsweise wenigstens eine pH-Einheit unterhalb des pKs-Wertes wenigstens einer der protonierbaren Gruppen der nukleinsäurebindenden Phase. Dies hat wie oben dargelegt zur Folge, dass die Elution unter besonders schonenden Bedingungen durchgeführt werden kann.

Vorzugsweise erfolgt die Elution je nach eingesetzten nukleinsäurebindenden Liganden bzw. nukleinsäurebindenden Phase bei einem pH-Wert von 7,5 bis 11, von 7,5 bis 10, vorzugsweise bei einem pH-Wert von 8 bis 9 bzw. 8,2 bis 8,8. Bei diesen pH-Werten werden besonders vorteilhafte Ergebnisse erzielt, da die Nukleinsäuren besonders schonend freigesetzt werden.

Um die direkte Weiterverarbeitung der isolierten Nukleinsäuren im Elutionspuffer zu ermöglichen, weist dieser vorzugsweise eine niedrige Salzkonzentration auf. Die Salzkonzentration liegt daher gemäß einer Ausführungsform bei ≤ 1 M vorzugsweise bei ≤ 0,5 M, bei 5 0,25 M, bei ≤ 0,1 M, besonders bevorzugt bei ≤ 50 mM, ≤ 25 mM oder sogar ≤ 10 mM. Geeignete Salze können Halogenide, insbesondere Chloride, der Alkali und Erdalkalimetalle oder Ammonium, andere Salze von Mineralsäuren, Acetate, Borate sowie Verbindungen wie Tris, Bis-Tris sowie auf der Basis organischer Puffer, wie bspw. MES, CHAPS, HEPES und ähnliche darstellen. Geeignete Substanzen zur Elution sind darüber hinaus im Stand der Technik bekannt.

Insbesondere bei der vakuum- oder zentrifugationsbasierten Elution der Nukleinsäuren bspw. bei Einsatz einer nukleinsäurebindenden Membran oder bei Einsatz von nukleinsäurebindenden Partikeln in Kombination mit einer Membran, welche als Barriere für die Partikel dient, kann es insbesondere bei kleinen Elutionsvolumina von Vorteil sein, wenn zusätzliche Maßnahmen ergriffen werden, die sicherstellen, dass innerhalb geringer Schwankungen auch jeweils das gleiche Elutionsvolumen erzielt wird. Dies insbesondere wenn mit Mehrfachansätzen wie bspw. einer 48 oder 96er Platte gearbeitet wird. So hat es sich als vorteilhaft erwiesen, auf die Elutionslösung wenigstens einen Kohlenwasserstoff zu geben. Dadurch wird in vorteilhafter Weise ein gleichmäßigeres Eluatvolumen erhalten. Der Kohlenwasserstoff und/ oder das Gemisch aus Kohlenwasserstoffen, können eines oder mehrere der nachfolgenden Merkmale aufweisen:

a) der Kohlenwasserstoff ist ein unsubstituiertes oder substituiertes Alkan;
b) der Kohlenwasserstoff ist ein mit Wasser nicht mischbares Alkan;
c) der Kohlenwasserstoff ist ein acyclisches Alkan;
d) der Kohlenwasserstoff ist ein unverzweigtes acyclisches Alkan;
e) der Kohlenwasserstoff ist ein verzweigtes acyclisches Alkan;
f) der Kohlenwasserstoff ist ein cyclisches Alkan;
g) der Kohlenwasserstoff ist ein Alkan mit 6 bis 16 Kohlenstoffatomen;
h) der Kohlenwasserstoff ist ein Alkan mit 8 bis 12 Kohlenstoffatomen;
i) der Kohlenwasserstoff ist ein Alkan, das ausgewählt ist aus der Gruppe n-Octoan, n-Nonan, n-Decan und n-Dodecan; und/oder
j) der Kohlenwasserstoff ist vorzugsweise ein Mineralöl.

Um die Aufreinigung zu begünstigen, wird nach der Bindung und vor der Elution der Nukleinsäuren vorzugsweise wenigstens ein Waschschritt durchgeführt. Zum Waschen sind wässrige Lösungen mit niedrigen Salzkonzentrationen und aber auch Wasser bevorzugt. Wenn Salze in den Waschpuffern enthalten sind, so bevorzugt in einer Konzentration von ≤ 400 mM, besonders bevorzugt von ≤ 200 mM, ≤ 100 mM, ≤ 50 mM und/oder sogar ≤ 25 mM. Organische Bestandteile können in dem Waschpuffer enthalten sein, so bspw. Alkohole, Polyole, Polyethylenglykole, Aceton, Acetonitril, Kohlenhydrate oder Mischungen derselben. Die Waschpuffer können jedoch ohne störende Mengen der entsprechenden organischen Bestandteile vorliegen, so dass nachfolgende Anwendungen, wie bspw. enzymatische Verarbeitungen, Amplifikationsreaktionen und dergleichen ("downstream" Applikationen) nicht behindert werden.

Die nukleinsäurebindenden Liganden werden mit dem Träger kovalent oder durch elektrostatische, polare oder hydrophobe Wechselwirkungen verknüpft. Vorzugsweise erfolgt dies im Rahmen einer Beschichtung. Sofern das Trägermaterial keine geeigneten funktionellen Gruppen zur Anbindung der nukleinsäurebindenden Liganden aufweist, kann zunächst eine Funktionalisierung des Trägermaterials erfolgen, um dieses mit geeigneten funktionellen Gruppen, insbesondere Carboxygruppen, auszustatten. Hierbei kann das entsprechende Profil der Beschichtung des Trägermaterials mit nukleinsäurebindenden Liganden im Unterschuss auch dadurch erzielt werden, dass das Trägermaterial entsprechend mit weniger funktionellen Gruppen zur Anbindung der nukleinsäurebindenden Liganden ausgestattet wird. Geeignete Beschichtungsverfahren sind im Stand der Technik bekannt und bedürfen daher keiner näheren Beschreibung. Die Bindung der nukleinsäurebindenden Liganden an den Träger und insbesondere an die funktionellen Gruppen des Trägers kann auf verschiedene Arten erfolgen. Die nukleinsäurebindenden Liganden können bspw. im Fall von Di - oder Polyaminen über eine Aminogruppe verbunden werden. Die nukleinsäurebindenden Liganden können jedoch auch eine funktionelle Gruppe aufweisen, die zur Anbindung an den Träger genutzt wird. Geeignete funktionelle Gruppen sind im Stand der Technik bekannt. Ferner besteht auch die Möglichkeit, die nukleinsäurebindenden Liganden direkt mit der Oberfläche des Trägers, vorzugsweise eines Polymerträgers zu verknüpfen. Dies kann bspw. direkt über das Kohlenstoffgerüst erfolgen.

Bevorzugte protonierbare Gruppen, die sich für die Bindung von Nukleinsäuren bewährt haben, sind Aminogruppen, bevorzugt sind primäre, sekundäre und tertiäre Aminogruppen. Vorzugsweise weisen die protonierbaren Gruppen, wie insbesondere die Aminogruppen einen pKs-Wert von wenigstens 8 auf. Bevorzugt liegt der pKs-Wert bei 9 bis 12, vorzugsweise bei 10 bis 12. Die nukleinsäurebindenden Liganden weisen vorzugsweise 1 bis 2 Aminogruppen auf. Bevorzugte nukleinsäurebindende Liganden sind bspw. primäre, sekundäre und tertiäre Mono- und Diamine. Diese können substituiert oder unsubstituiert sein.

Bevorzugte Beispiele für nukleinsäurebindende Liganden sind insbesondere primäre, sekundäre und tertiäre Amine der Formeln

(a) $R_3N$
(b) $R_2NH$
(c) $RNH_2$ und/oder
(d) $X\text{-}(CH_2)_n\text{-}Y$ mit $X = R_2N$, $RNH$ oder $NH_2$ und $Y = R_2N$ oder $RNH$ oder $NH_2$

wobei
R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
n= 0 bis 20

Bevorzugt wird N-Propyl-1,3-propandiamin als nukleinsäurebindender Ligand eingesetzt.

Darüber hinaus können auch ringförmige Amine, aromatische Amine oder aminofunktionalisierte Heterozyklen eingesetzt werden. Die Amine können Substituenten tragen, bspw. Alkyl-, Alkenyl-, Alkinyl- oder aromatische Substituenten, darüber hinaus können die Kohlenwasserstoffketten auch zu einem Ring geschlossen sein. Die Kohlenwasserstoffketten können auch Heteroatome, wie Sauerstoff, Stickstoff, Schwefel oder Silizium, oder Verzweigungen aufweisen. Die Aminogruppen der Amine weisen vorzugsweise pKs-Werte von 9 bis 12, besonders bevorzugt von 10 bis 12 auf.

Weitere geeignete nukleinsäurebindende Liganden sind Polyoxyalkylenamine mit einer, zwei oder drei Aminogruppen. Diese sind beispielsweise erhältlich unter dem Namen "Jeffamine" Polyoxylalkylenamine. Jeffamine enthalten primäre Aminogruppen, die an den Terminus des Polyetherbackbones gebunden sind. Das Polyetherbackbone kann auf Propylenoxid, Ethylenoxid oder Mischungen daraus basieren; auch ist der Einsatz anderer Backbonesegmente denkbar.

Auch Mischungen der entsprechenden nukleinsäurebindenden Liganden können erfindungsgemäß eingesetzt bzw. auf einen Träger aufgebracht werden.

Vorzugsweise stehen die Aminogruppen der nukleinsäurebindenden Liganden nicht in Konjugation mit einer die Elektronendichte verringernden Gruppe wie bspw. einer Carboxylgruppe, einer Carbonylgruppe, einer Gruppe mit C-C-Doppelbindungen oder einer β-Hydroxyethylgruppe, so dass ihr pKs-Wert vorzugsweise zwischen 9 und 12 liegt. Eine Konjugation mit einer Elektronendichte verringernden Gruppe gilt dann als vorhanden, wenn eine Aminofunktion und die entsprechende, die Elektronendichte verringernde Gruppe über nur drei, zwei oder weniger Kohlenstoffatome verbunden sind.

Als Träger für die nukleinsäurebindenden Liganden kommen bspw. organische Polymere wie Polystyrol und seine Derivate, Polyacrylate und -methacrylate, und ihre Derivate oder Polyurethane, Nylon, Polyethylen, Polypropylen, Polybutylen, und Copolymere aus diesen Materialien infrage. Darüber hinaus können diese nukleinsäurebindenden Liganden auch mit Polysacchariden, insbesondere Hydrogelen wie Agarose, Cellulose, Dextran, Sephadex, Sephacryl, Chitosan verknüpft sein. Des weiteren können die nukleinsäurebindenden Liganden auch an anorganische Träger wie bspw. Glas oder weitere Metall- und Halbmetalloxide, Boroxid oder Metalloberflächen, wie z.B. Gold, angebunden werden. Vorzugsweise weist das Trägermaterial keine eigene Nukleinsäurebindekapazität auf.

Magnetische Partikel sind besonders vorteilhaft in der Handhabung. Die nukleinsäurebindende Phase ist daher vorzugsweise magnetisch und kann paramagnetisch, ferrimagnetisch, ferromagnetisch oder superparamagnetisch sein. Bevorzugt sind paramagnetische Partikel. Die einzelnen Schritte und Ausführungsvarianten zur Handhabung von magnetischen Partikeln wie sie erfindungsgemäß bevorzugt zum Einsatz kommen, sind im Stand der Technik wohl bekannt und benötigen daher ebenfalls keine genaue Beschreibung.

Die nukleinsäurebindenden Liganden können direkt oder aber über Abstandshalter, sogenannte "Spacer", an diese Träger gebunden sein. Sie können auch Teil eines größeren Moleküls sein. Beispiele für Spacer sind Kohlenwasserstoffketten, Poylethylen- oder Polypropylenglykole, sowie funktionalisierte Silane. Diese Spacer können verzweigt oder unverzweigt vorliegen.

Als chemische Funktionalitäten für die Anbindung der nukleinsäurebindenden Liganden können Säureamide oder - anhydride, Epoxide, Tosylgruppen, Formylgruppen, Sulfonylchloride, Maleinimide oder mit Carbodiimidchemie aktivierte Carboxylatgruppen eingesetzt werden. Im Rahmen der Erfindung ebenfalls möglich ist eine nichtkovalente Anbindung der nukleinsäurebindenden Liganden wie bspw. Amine, z.B. über ionische Wechselwirkungen oder über adsorptive Prozesse. Die nukleinsäurebindenden Liganden können auch über Thiole an z.B. Goldoberflächen angebunden sein. Bevorzugt ist die Anbindung der nukleinsäurebindenden Liganden an carboxylierte Oberflächen.

Weitere Ausführungsformen der Trägermaterialien umfassen nichtmagnetische und magnetische Partikel, Säulenmaterialien, Membranen, sowie Oberflächenbeschichtungen. Ferner seien funktionalisierte Träger wie Röhrchen, Membrane, Vliese, Papier, Reaktionsgefäße wie PCR-Gefäße, "Eppendorf-Tubes", Multiplates, Chips und Mikroarrays genannt.

Besonders entscheidend für den Wirkungsgrad der Nukleinsäureaufreinigung ist eine effiziente Elution und damit Ablösung der gebundenen Nukleinsäuren von der nukleinsäurebindenden Phase. Hier wurde überraschend festgestellt, dass nicht nur die pKs-Werte der protonierbaren Gruppen der nukleinsäurebindenden Liganden entscheidend sind. Auch die Struktur der nukleinsäurebindenden Phase und die Anwesenheit anderer funktioneller Gruppen tragen dazu bei, die Elution bei pH-Werten im neutralen bzw. schwach alkalischen Bereich zu begünstigen und zu verbessern.

Gemäß einer Ausführungsform trägt die nukleinsäurebindende Phase zusätzlich funktionelle Gruppen, die beim Elutions pH-Wert die Elution der Nukleinsäuren fördern, bspw. indem sie beim Elutions pH-Wert einen abstoßenden Effekt ausüben. Vorzugsweise liegen diese funktionellen Gruppen daher bei der Elution negativ geladen vor. Die pKs-Werte dieser Gruppen können bspw. in einem Bereich von 0 bis 7, vorzugsweise 1 bis 5 liegen. Geeignet sind bspw. Ionenaustauscher, insbesondere Kationenaustauscher, bevorzugt saure Gruppen wie bspw. Carboxylgruppen. Weitere geeignete Gruppen sind Betaine, Sulfonate, Phosphonate und Phosphate. Bspw. kann der feste Träger mit Carboxylgruppen funktionalisiert sein, um eine Anbindung der nukleinsäurebindenden Liganden zu ermöglichen. Bei der Anbindung der nukleinsäurebindenden Liganden kann die Konzentration selbiger so gewählt sein, dass einige der Carboxylgruppen frei vorliegen und daher nicht mit den nukleinsäurebindenden Liganden funktionalisiert werden. Diese behindern bei niedrigen pH-Werten die Bindung der Nukleinsäuren nicht. Bei höheren pH-Werten liegen diese jedoch vorzugsweise negativ geladen vor und begünstigen dadurch die Ablösung der Nukleinsäuren von den nukleinsäurebindenden Liganden. Diese Wechselwirkung kann ferner durch die Auswahl der Länge bzw. des Abstandes zwischen den protonierbaren Gruppen der nukleinsäurebindenden Liganden und den negativ ionisierbaren Gruppen, wie bspw. den Carboxylgruppen begünstigt werden. Dadurch wird in vorteilhafter Weise die Elution bei niedrigen pH-Werten begünstigt, so dass die Ausbeute steigt. Die Auswahl, Stärke und Länge der funktionellen Gruppen, die bei dem Elutions pH-Wert einen abstoßenden Effekt auf die Nukleinsäuren ausüben, variiert je nach ausgewählter nukleinsäurebindender Gruppe, so insbesondere der Anzahl der protonierbaren Gruppen pro nukleinsäurebindender Gruppe und deren Abstand zu den die Elution fördernden funktionellen Gruppen.

Durch Wahl/Kombination der beschriebenen Parameter, insbesondere der die Elution fördernden funktionellen Gruppen, der Verdünnungsliganden, sowie der Verdünnung bzw. Mischung mit nukleinsäurebindenden Liganden kann der pH-Wert der nukleinsäurebindenden Phase in Bezug auf die Elutionsbedingungen optimal eingestellt werden. Entsprechend kann das Elutionsprofil der nukleinsäurebindenden Phase, insbesondere der Elutions pH-Wert gesteuert bzw. eingestellt werden.

Nukleinsäuren, die mit dem erfindungsgemäßen Verfahren aufgereinigt werden können, können in Körperflüssigkeiten wie Blut, Urin, Stuhl, Speichel, Sputum, oder sonstigen Körperflüssigkeiten, in biologischen Quellen wie Gewebe, Zellen, insbesondere Tierzellen, Humanzellen, Pflanzenzellen, Bakterienzellen und dergleichen, Organen wie Leber, Niere oder Lunge etc. vorliegen. Dazu kann die Nukleinsäure aus Trägermaterialien wie Swabs, PapSmears, sowie stabilisierenden Medien wie PreServCyt oder Surepath, oder auch aus weiteren Flüssigkeiten, wie z.B. Säften, wässrigen Proben oder allgemein Lebensmitteln gewonnen werden. Darüber hinaus können die Nukleinsäuren aus Pflanzenmaterial, bakteriellen Lysaten, in Paraffin eingebettetem Gewebe, wässrige Lösungen oder Gelen gewonnen werden.

Sofern die Nukleinsäure in einem Zellmaterial vorliegt, kann das Zellmaterial wie im Stand der Technik bekannt zerstört und insbesondere lysiert werden, um die Nukleinsäuren freizusetzen. Entsprechende Zellaufbruchsverfahren bzw. Lyseverfahren sind im Stand der Technik bekannt und benötigen daher keine weitere Beschreibung.

Aufgrund der Tatsache, dass man bei dem erfindungsgemäßen Verfahren auf Wasser basierende Puffer einsetzen und auf gefährliche Chemikalien verzichten kann, werden sehr saubere Eluate erzielt, die vorzugsweise keine Substanzen enthalten, die für die downstream Applikationen wie bspw. PCR, Restriktionsverdau, Transfektion oder Sequenzreaktionen nachteilig sind. Darüber hinaus ist es mit der erfindungsgemäßen Methode möglich, unter sehr schonenden Bedingungen, wie bspw. pH 6,5 - 7 die Nukleinsäure zu binden und nur bei leicht alkalischem pH wie 8,5 und sehr niedrigen Salzkonzentrationen wie zum Beispiel 25 mmol Tris zu eluieren, so dass für die weiteren Untersuchungen und Anwendungen (downstream Applikationen) keine Verdünnung oder Neutralisation notwendig ist.

Die erfindungsgemäß standardisierte bzw. normalisierte Nukleinsäurekonzentration kann bevorzugt für eine enzymatische Nukleinsäureamplifikation, Modifikationsreaktion oder Sequenzierreaktionen eingesetzt werden. Die erfindungsgemäß aufgereinigten und damit normalisierten Nukleinsäuren eignen sich auch bspw. für Short Tandem Repeat (STR), Analysen und Transfektionen, bei denen ebenfalls definierte Mengen von Nukleinsäuren zum Einsatz kommen.

Darüber hinaus betrifft die vorliegende Erfindung die Verwendung einer nukleinsäurebindenden Phase wie oben beschrieben zur Aufreinigung einer definierten Menge von Nukleinsäuren aus einer Probe und damit zur Normalisierung der Nukleinsäurekonzentration. Die nukleinsäurebindende Phase weist nukleinsäurebindende Liganden mit wenigstens einer protonierbaren Gruppe auf, wobei die nukleinsäurebindenden Liganden an einen Träger gebunden vorliegen und die nukleinsäurebindende Phase eine Oberfläche mit einer niedrigen Ladungsdichte aufweist, wobei ein Elutions pH-Wert eingestellt wird, der oberhalb des Bindungs pH-Wertes liegt und wobei die Nukleinsäuren in der Probe im Überschuss zu der Bindungskapazität der eingesetzten Menge an nukleinsäurebindender Phase vorliegen.

Die niedrige Ladungsdichte wird vorzugsweise durch eines oder mehrere der nachfolgenden Merkmale erzielt:

a) die nukleinsäurebindenden Liganden weisen am Träger gebunden jeweils nicht mehr als ein oder zwei protonierbare Gruppen zur Bindung der Nukleinsäuren auf; und/oder
b) die nukleinsäurebindenden Gruppen sind ausgewählt aus der Gruppe der Mono- und Diamine; und/oder
c) der Träger ist mit einer Mischung aus nukleinsäurebindenden Gruppen und Verdünnungsgruppen beschichtet; und/oder
d) die nukleinsäurebindenden Liganden liegen im Unterschuss an den Träger gebunden vor.

Die nukleinsäurebindende Phase, die erfindungsgemäß zum Einsatz kommt, weist insbesondere nukleinsäurebindende Liganden mit wenigstens einer protonierbaren Gruppe auf, die einen pKs-Wert von wenigstens 8, vorzugsweise 9 bis 12 aufweist. Bevorzugte Ausgestaltungen sind oben im Detail beschrieben (siehe obige Offenbarung) und insbesondere durch ein oder mehrere der folgenden Merkmale gekennzeichnet:

a. die protonierbaren Gruppe(n) weisen einen pKs-Wert von 9, bis 12 vorzugsweise einen pKs-Wert von 10 bis 12 auf; und/oder
b. die protonierbaren Gruppen sind Aminogruppen und liegen nicht in Konjugation mit die Elektronendichte verringernden Gruppen vor; und/oder
c. die nukleinsäurebindende Phase weist funktionelle Gruppen auf, die bei dem Elutions pH-Wert die Freisetzung/ Elution der Nukleinsäuren begünstigen, vorzugsweise Kationenaustauscher, insbesondere Carboxylgruppen; und/ oder
d. der Träger weist keine eigenen nukleinsäurebindenden Eigenschaften neben den nukleinsäurebindenden Liganden auf; und/oder
e. der Träger ist ausgewählt aus der Gruppe bestehend aus organischen Polymeren wie Polystyrol und seinen Derivaten, Polyacrylaten und -methacrylaten und ihren Derivaten, Polyurethanen, Nylon, Polyethylen, Polypropylen, Polybutylen und Copolymeren aus diesen Materialien, Polysacchariden und Hydrogelen wie Agarose, Cellulose, Dextran, Sephadex, Sephacryl, Chitosan, anorganischen Trägern, Glas oder weiteren Metall- und Halbmetalloxiden, Boroxid, Trägern mit Metalloberflächen, wie z. B. Gold, magnetische Partikel oder Mischungen derselben; und/oder
f. die nukleinsäurebindenden Liganden sind ausgewählt aus der Gruppe bestehend aus primären, sekundären und tertiären Aminen der Formeln

(a) $R_3N$
(b) $R_2NH$
(c) $RNH_2$ und/oder
(d) $X\text{-}(CH_2)_n\text{-}Y$ mit $X = R_2N$, $RNH$ oder $NH_2$ und $Y = R_2N$ oder $RNH$ oder $NH_2$

wobei
R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
n= 0 bis 20,
und stellen vorzugsweise Mono- und Diamine dar. Diese können substituiert oder unsubstituiert sein.

Ferner wird mit der Erfindung ein Kit zur Aufreinigung einer definierten Menge von Nukleinsäuren zur Verfügung gestellt, bei dem eine erfindungsgemäße nukleinsäurebindende Phase eingesetzt wird. Einzelheiten zu der nukleinsäurebindenden Phase und den Vorteilen in Bezug auf die Normalisierung sind oben im Detail beschrieben und gelten auch im Zusammenhang mit dem erfindungsgemäßen Kit. Wir verweisen auf die obige Offenbarung.

Das erfindungsgemäße Kit weist vorzugsweise wenigstens eines der folgenden Merkmale auf:

a. einen Bindungspuffer mit einem pH-Wert, der wenigstens eine pH Einheit unterhalb des pKs-Wertes wenigstens einer der protonierbaren Gruppen der nukleinsäurebindenden Phase liegt, und/oder einen Bindungspuffer, der die Einstellung eines solchen pH-Wertes in der Probe ermöglicht; und/oder

b. einen Elutionspuffer mit einem pH-Wert, der eine pH Einheit unterhalb des pKs-Wertes wenigstens einer der protonierbaren Gruppen der nukleinsäurebindenden Phase liegt, und/oder einen Elutionspuffer, der die Einstellung eines solchen pH-Wertes in der Probe ermöglicht.

Gemäß einer Ausführungsform weist das Kit einen Bindungspuffer auf, der wenigstens eines der folgenden Merkmale aufweist:

i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von $\leq$ 1M, $\leq$ 0,5M, $\leq$ 0,25M oder w$\leq$ 0,1M;

Das Kit kann ferner einen Elutionspuffer aufweisen, der wenigstens eines der folgenden Merkmale aufweist:

i. einen pH-Wert von 7,5 bis 10; und/oder
ii. einen pH-Wert von 8 bis 9; und/oder
iii. einen pH-Wert von 8,2 bis 8,8; und/oder
iv. eine Salzkonzentration von $\leq$ 1M, $\leq$ 0,5M, $\leq$ 0,25M, $\leq$ 0,1M, $\leq$ 25mM, $\leq$ 15mM oder $\leq$ 10mM; und/oder
v. er ist ausgewählt aus der Gruppe bestehend aus Wasser, biologischem und organischem Puffer.

Einzelheiten zur nukleinsäurebindenden Phase sowie den Bindungs- und Elutionsbedingungen sind im Detail oben beschrieben und gelten auch im Zusammenhang mit dem erfindungsgemäßen Kit und kennzeichnen die darin verwendeten Bestandteile/Puffer. Wir verweisen auf die obige Offenbarung. Darüber hinaus kann das Kit weitere übliche Bestandteile enthalten, so bspw. Lyse-, Wasch - und/oder Neutralisierungsreagenzien bzw. Puffer.

Die entsprechenden Kits können insbesondere im Rahmen des erfindungsgemäßen Verfahrens zur Anwendung kommen und sind besonders zur Normalisierung geeignet. Daher kann das erfindungsgemäße Kit insbesondere bei automatisierten Systemen zum Einsatz kommen.

Die vorliegenden Verfahren, Kits sowie nukleinsäurebindenden festen Phasen können insbesondere im Bereich der Molekularbiologie, der molekularen Diagnostik, in der Forensik, in der Lebensmittelanalytik sowie im Applied Testing eingesetzt werden. Sie sind insbesondere für die automatisierte Anwendung geeignet.

Die eluierten Nukleinsäuren können vorzugsweise direkt weiterverarbeitet werden, so bspw. im Rahmen molekularbiologischer Anwendungen wie bspw. einer PCR, RT-PCR, einem Restriktionsverdau, einer Sequenzierung oder einer Transkription zum Einsatz kommen. Eine weitere Aufreinigung ist nicht erforderlich, sofern die Elutionspuffer wie oben beschrieben ausgestaltet sind und vorzugsweise eine niedrige Salzkonzentration aufweisen.

Als Nukleinsäuren kommen für eine Aufreinigung DNA und RNA in Frage, insbesondere genomische DNA, Plasmid-DNA, sowie PCR Fragmente, cDNA, miRNA, siRNA, sowie Oligonukleotide und modifizierte Nukleinsäuren wie bspw. PMA oder LMA. Es können auch virale oder bakterielle RNA sowie DNA oder Nukleinsäuren aus menschlichen, tierischen oder pflanzlichen Quellen aufgereinigt werden. Des Weiteren kommen für eine erfindungsgemäße Aufreinigung auch DNA/RNA Hybride in Frage, um nur einige Beispiele zu nennen.

Die vorliegende Erfindung soll nachfolgend anhand von einigen Beispielen erläutert werden. Diese sind nicht beschränkend, stellen jedoch bevorzugte Ausführungsformen der vorliegenden Erfindung dar. Darüber hinaus werden sämtliche der hierin genannten Referenzen zum Gegenstand der Offenbarung gemacht.

**BEISPIELE**

Im Rahmen der Experimente wurden als Modellsysteme Long Range (LR) PCR Fragmente (8 bis 11kb) eingesetzt. Die Versuche wurden anhand der nachfolgenden Vorschriften durchgeführt.

**Fig. 1** zeigt die Abhängigkeit der eluierten DNA-Menge (LR PCR Fragmente) von der Ausgangs-DNA Menge bei Einsatz von mit Spermin beschichteten Polymerbeads. Je mehr DNA den mit Polyaminen beschichteten Partikeln angeboten wurden, desto mehr DNA konnte gebunden und dann wieder eluiert werden. Ein Plateau der Nukleinsäurebindekapazität wird jedoch selbst bei größeren Nukleinsäuremengen in der Probe nicht erreicht, eine Normalisierung trat auch bei Einsatz verschiedener Ausgangs-DNA Mengen nicht ein.

**A) Umsetzung von magnetischen Polymeren mit Aminen**

Materialien

Als magnetisches Polymer können verschiedene Materialien eingesetzt werden, darunter Estapor, Dynal, Seradyn oder Ademtech Partikel.

Amin: N-Propyl-1,3-propandiamin (Aldrich, Katalog-Nr. 308153)

Herstellvorschrift

Man resuspendiert 500 mg carboxylierte Magnetpartikel in 10 ml MES Puffer und setzt dann 10 ml einer 50 mg/ml Lösung von N-Hydroxysuccinimid zu. Man mischt mit einem Minishaker, setzt dann 10 ml einer 50 $\mu$mol/l Lösung von 1-Ethyl-3-(3-dimethylaminopropyl)carbodiimid zu und vortext erneut. Anschließend lässt man 30 Minuten am Überkopfschüttler reagieren und trennt dann den Überstand ab. Man resuspendiert in 50 ml MES Puffer. Die Suspension wird magnetisch separiert und der Überstand verworfen. Dann nimmt man in 5 ml MES Puffer auf und setzt 10 ml einer wässrigen 1,4-Diaminobutan Lösung zu (100 mg/ml in MES, pH-Wert 8,5), vortext gründlich und lässt eine Stunde am Überkopfschüttler reagieren. Anschließend wäscht man zweimal mit je 50 ml MES Puffer, separiert magnetisch und verwirft die Überstände. Die Partikel werden dann in 10 ml MES-Puffer resuspendiert.

**B) Normalisierung von LR-PCR Fragmenten**

**1) Normalisierung unterschiedlicher Ausgangs-DNA-Mengen derselben Probe**

Waschen der Beads:

4 Ansätze: je 2$\mu$l Beads mit 2x100$\mu$l 50mM TrisHCl pH6,5 waschen

Die Konzentrierung der Beads erfolgt jeweils für mindestens 30 sec an einem Magneten, bevor der Überstand abgenommen wird.

Bindung der DNA:

Suspendieren der Beads in 100$\mu$l Waschpuffer (s.o.) + 2$\mu$l 3M NaAc pH5,3

| | | | |
|---|---|---|---|
| **1** | + 4$\mu$l DNA LR-PCR06 | (8 kb Fragment) | |
| **2** | + 3$\mu$l DNA LR-PCR06 | + 1$\mu$l LR-PCR-Puffer 1x | |
| **3** | + 2$\mu$l DNA LR-PCR06 | + 2$\mu$l LR-PCR-Puffer 1x | |
| **4** | + 1$\mu$l DNA LR-PCR06 | + 3$\mu$l LR-PCR-Puffer 1x | |

10min bei RT schütteln

Waschen:

2x Waschen mit 100$\mu$l Wasser

Elution:

+ 20$\mu$l 50mM Tris-HCl pH 8,5, 50mM NaCl
5 min RT
Überführen des Überstand in neue Gefäße.

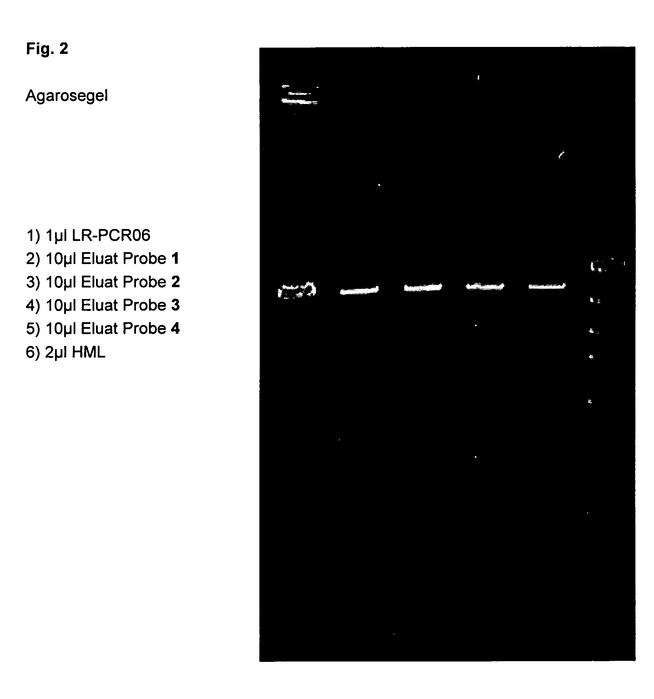

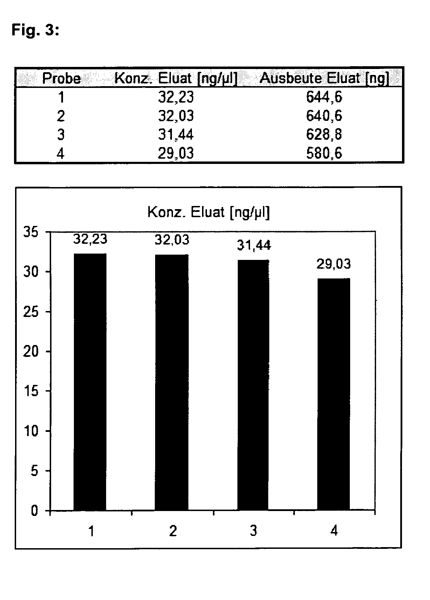

Die Ergebnisse der Normalisierung sind in den **Fig. 2** und **3** zusammengefasst.

**2) Normalisierung unterschiedlicher DNA-Fragmente zwischen 8 und 11kb**

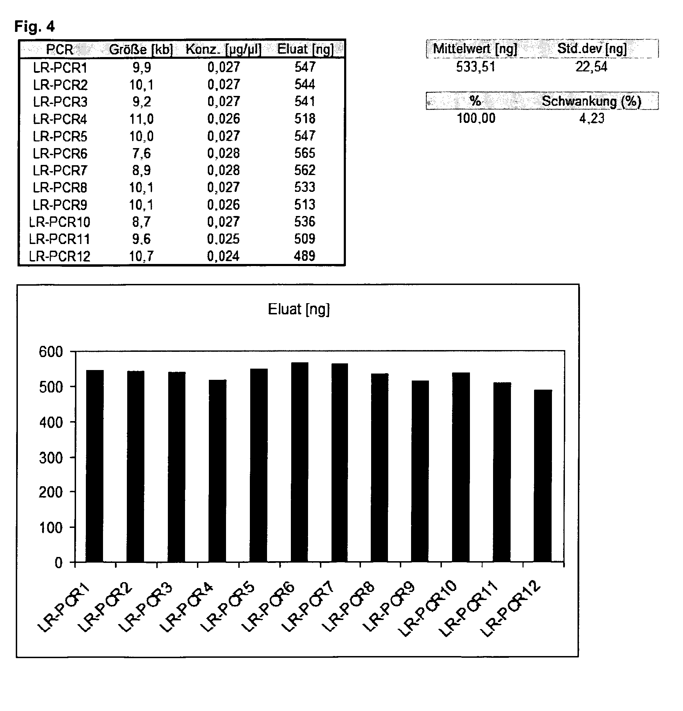

12 unterschiedliche Long Range PCR-Proben wurden nach dem Protokoll gemäß 1) aufgereinigt und normalisiert. **Fig. 4** fasst die DNA-Mengen im Eluat nach Pico-Green-Messung zusammen.

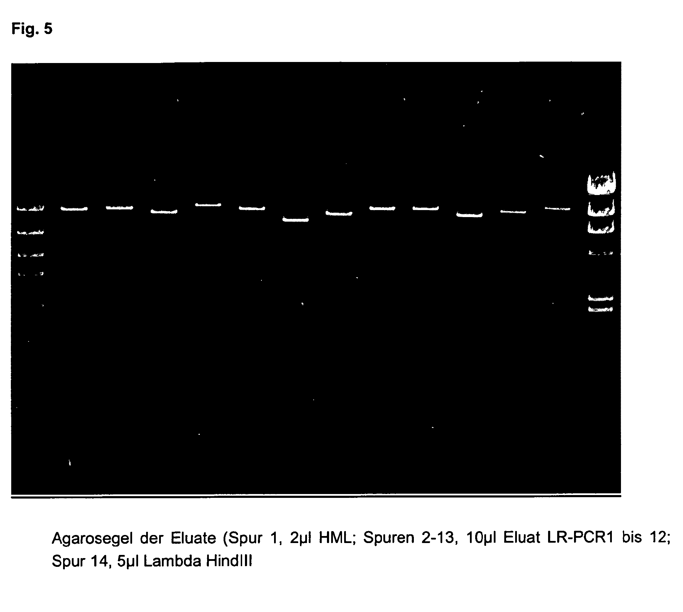

Anschließend wurden die 12 Proben zusammengefasst und die durchschnittliche Konzentration durch $OD_{260nm}$ Messung kontrolliert. Hier wurde ein durchschnittlicher Wert von 580ng pro PCR-Fragment bestimmt, was mit dem Durchschnittswert nach PicoGreen-Messung von 533ng vergleichbar ist. **Fig. 5** zeigt die Ergebnisse. Die schwächeren Banden in den Spuren 12 und 13 lassen sich durch einen erhöhten DNA-Hintergrund, der durch unspezifische Amplifikation verursacht wurde, erklären.

In verschiedenen Versuchen, konnte gezeigt werden, dass Fragmente ähnlicher Größe (8-11kb) und unterschiedlicher Größe (z.B. 1kb, 5kb, 10kb; Ergebnisse nicht dargestellt) mit geringen Schwankungen unter 10% normalisiert werden konnten. Damit stellt das hier vorgestellte Verfahren eine deutliche Verbesserung gegenüber den bisher bekannten Verfahren der Normalisierung dar, deren Schwankungen in den Ausbeuten von 50% bzw. 2-3fach deutlich größer sind.

Wenn das vorliegende Verfahren zur Normalisierung von PCR-Fragmenten eingesetzt wird, so ist durch die Kenntnis der Fragmentlängen neben der Normalisierung der DNA-Massen auch eine Normalisierung der Fragmentmengen (Molarität) mittels der Formel

$$Volume[\mu l]=MW[\mu g/\mu mol]/conc[\mu g/\mu l]$$

möglich.

Das erfindungsgemäße Verfahren hat daher erhebliche Vorteile gegenüber dem Stand der Technik. So wird eine sehr gute Ausbeute von 500 ng pro 2 $\mu$l Beads in 20 $\mu$l Eluat erreicht, wobei Schwankungen von lediglich +/- 10 % auftreten. Das erfindungsgemäße Verfahren kann automatisiert und mit magnetischen Separatoren, Pipetten und Shakern eingesetzt werden. Darüber hinaus ist es besonders zeiteffizient, da innerhalb von 20 Minuten 96 Proben in einem automatisierten Verfahren aufgereinigt werden können. Dies ist ein erheblicher Vorteil gegenüber dem Stand der Technik, wo teilweise 60 Minuten und mehr für die Aufreinigung der gleichen Probenmenge benötigt werden.

**Patentansprüche**

**1.** Verfahren zur Isolierung und/oder Aufreinigung einer definierten Menge von Nukleinsäuren aus einer nukleinsäurehaltigen Probe, das wenigstens die nachfolgenden Schritte aufweist:

    a. Inkontaktbringen der nukleinsäurehaltigen Probe mit einer definierten Menge einer nukleinsäurebindenden Phase mit folgenden Merkmalen:

        (i) die nukleinsäurebindende Phase weist nukleinsäurebindende Liganden auf, die wenigstens eine protonierbare Gruppe aufweisen;
        (ii) die nukleinsäurebindenden Liganden liegen an einen Träger gebunden vor;
        (iii) die nukleinsäurebindende Phase weist eine Oberfläche mit einer niedrigen Ladungsdichte auf,

    wobei die Menge an Nukleinsäuren in der Probe die Bindungskapazität der eingesetzten Menge an nukleinsäurebindender Phase übersteigt;
    b. Bindung der Nukleinsäuren an die nukleinsäurebindende Phase bei einem pH-Wert (Bindungs pH-Wert), der unterhalb des pKs-Wertes wenigstens einer der protonierbaren Gruppen liegt;
    c. Elution der Nukleinsäuren bei einem pH-Wert, der oberhalb des Bindungs pH-Wertes liegt, wobei eine definierte Menge an Nukleinsäuren erhalten wird.

**2.** Das Verfahren nach Anspruch 1, **dadurch gekennzeichnet, dass** die niedrige Ladungsdichte durch eines oder mehrere der nachfolgenden Merkmale erzielt wird:

    a. die nukleinsäurebindenden Liganden weisen am Träger gebunden jeweils nicht mehr als ein oder zwei protonierbare Gruppen zur Bindung der Nukleinsäuren auf; und/oder
    b. die nukleinsäurebindenden Liganden sind ausgewählt aus der Gruppe der Mono- und Diamine; und/oder

c. der Träger ist mit einer Mischung aus nukleinsäurebindenden Liganden und Verdünnungsliganden beschichtet; und/oder

d. die nukleinsäurebindenden Liganden liegen im Unterschuss an den Träger gebunden vor.

3. Das Verfahren nach Anspruch 1 oder 2, **dadurch gekennzeichnet, dass** die protonierbaren Gruppe(n) eines oder mehrere der nachfolgenden Merkmale aufweisen:

    a. einen pKs-Wert von 9 bis 12;
    b. einen pKs-Wert von 10 bis 12;
    c. die protonierbaren Gruppen sind Aminogruppen und liegen nicht in Konjugation mit die Elektronendichte verringernden Gruppen vor.

4. Das Verfahren nach einem oder mehreren der Ansprüche 1 bis 3, **dadurch gekennzeichnet, dass** die nukleinsäurebindende Phase wenigstens eines oder mehrere der nachfolgenden Merkmale aufweist:

    a. der Träger und/oder die nukleinsäurebindenden Liganden weisen funktionelle Gruppen auf, die beim Elutions pH-Wert die Freisetzung der Nukleinsäuren fördern;
    b. die nukleinsäurebindenden Liganden sind ausgewählt aus der Gruppe bestehend aus primären, sekundären und tertiären Aminen der Formeln

$$R_3N, R_2NH, RNH_2 \text{ und/oder } X\text{-}(CH_2)_n\text{-}Y$$

    mit
    $X = R_2N$, RNH oder $NH_2$
    $Y = R_2N$ oder RNH oder $NH_2$
    R = unabhängig voneinander ein verzweigter, unverzweigter oder ringförmiger Alkyl-, Alkenyl-, Alkinyl- oder aromatischer Substituent, wobei dieser auch ein oder mehrere Heteroatome enthalten kann;
    n= 0 bis 20, vorzugsweise 0 bis 18;
    c. der Träger weist keine eigenen nukleinsäurebindenden Eigenschaften neben den nukleinsäurebindenden Liganden auf;
    d. der Träger ist ausgewählt aus der Gruppe bestehend aus organische Polymere wie Polystyrol und seine Derivate, Polyacrylate und -methacrylate und ihre Derivate, Polyurethane, Nylon, Polyethylen, Polypropylen, Polybutylen und Copolymere aus diesen Materialien, Polysaccharide und Hydrogele wie Agarose, Cellulose, Dextran, Sephadex, Sephacryl, Chitosan, anorganische Träger, Glas oder weitere Metall- und Halbmetalloxide, Boroxid, Träger mit Metalloberflächen, wie z. B. Gold und magnetische Partikel; und/oder
    e. die Bindekapazität liegt in einem Bereich von 1 bis 500$\mu$g Nukleinsäure pro mg nukleinsäurebindender Phase, vorzugsweise 1 bis 200$\mu$g, besonders bevorzugt 10 bis 100$\mu$g Nukleinsäure pro mg nukleinsäurebindender Phase.

5. Das Verfahren nach Anspruch 4 (a), **dadurch gekennzeichnet, dass** Kationenaustauscher als funktionellen Gruppen vorliegen, insbesondere saure Gruppen wie vorzugsweise Carboxylgruppen.

6. Das Verfahren nach einem oder mehreren der Ansprüche 1 bis 5, **dadurch gekennzeichnet, dass** die Bindung unter Bedingungen erfolgt, die wenigstens eines der nachfolgenden Merkmale aufweisen:

    a. die Bindung erfolgt bei einem pH-Wert von 3 bis 8; und/oder
    b. die Bindung erfolgt bei einem pH-Wert von 4 bis 7,5; und/oder
    c. die Bindung erfolgt bei einem pH-Wert von 4,5 bis 7; und/oder
    d. die Bindung erfolgt bei einem pH-Wert von 5,5 bis 7; und/oder
    e. die Bindung erfolgt bei einem pH-Wert von 6,5 bis 7; und/oder
    f. die Bindung erfolgt bei einer Salzkonzentration von $\leq$ 1M, $\leq$ 0,5M, $\leq$ 0,25M oder $\leq$ 0,1M.

7. Das Verfahren nach einem oder mehreren der Ansprüche 1 bis 6, **dadurch gekennzeichnet, dass** die Elution unter Bedingungen erfolgt, die wenigstens eines der nachfolgenden Merkmale aufweisen:

    a. die Elution erfolgt bei einem pH-Wert, der oberhalb des Bindungs pH-Wertes liegt, jedoch wenigstens eine pH-Einheit unterhalb des pKs-Wertes wenigstens einer der protonierbaren Gruppen liegt; und/oder
    b. die Elution erfolgt bei einem pH-Wert von 7,5 bis 10; und/oder

c. die Elution erfolgt bei einem pH-Wert von 8 bis 9; und/oder

d. die Elution erfolgt bei einem pH-Wert von 8,2 bis 8,8; und/oder

e. die Elution erfolgt bei einer Salzkonzentration von $\leq$ 1M, $\leq$ 0,5M, $\leq$ 0,25M, $\leq$ 0,1M, $\leq$ 25mM, $\leq$ 15mM oder $\leq$ 10mM; und/oder

f. zur Elution wird eine Lösung eingesetzt, die ausgewählt ist aus der Gruppe von Wasser, biologischem und organischem Puffer.

8. Das Verfahren nach einem oder mehreren der Ansprüche 1 bis 7, **dadurch gekennzeichnet, dass** nach der Bindung und vor der Elution ein Waschschritt durchgeführt wird.

9. Das Verfahren nach Anspruch 8, **dadurch gekennzeichnet, dass** eine Waschlösung eingesetzt wird, die eines oder mehrere der folgenden Merkmale aufweist:

a. für den Waschschritt wird Wasser oder eine wässrige Lösung mit niedriger Salzkonzentration eingesetzt; und/oder

b. es wird eine wässrige Lösung mit niedriger Salzkonzentration eingesetzt, wobei die Salzkonzentration 5 400mM, $\leq$ 200mM, $\leq$ 100mM, $\leq$ 50mM und/oder $\leq$ 25mM beträgt.

10. Verwendung einer nukleinsäurebindenden Phase zur Isolierung und/oder Aufreinigung einer definierten Menge an Nukleinsäuren aus einer nukleinsäurehaltigen Probe, **dadurch gekennzeichnet, dass** die nukleinsäurebindende Phase nukleinsäurebindende Liganden mit wenigstens einer protonierbaren Gruppe aufweist, wobei die nukleinsäurebindenden Liganden an einen Träger gebunden vorliegen und die nukleinsäurebindende Phase eine Oberfläche mit einer niedrigen Ladungsdichte aufweist, wobei die niedrige Ladungsdichte durch ein oder mehrere der nachfolgenden Merkmale erzielt wird:

a. die nukleinsäurebindenden Liganden weisen am Träger gebunden jeweils nicht mehr als ein oder zwei protonierbare Gruppen zur Bindung der Nukleinsäuren auf; und/oder

b. die nukleinsäurebindenden Liganden sind ausgewählt aus der Gruppe der Mono- und Diamine; und/oder

c. der Träger ist mit einer Mischung aus nukleinsäurebindenden Liganden und Verdünnungsgruppen beschichtet; und/oder

d. die nukleinsäurebindenden Liganden liegen im Unterschuss an den Träger gebunden vor;

wobei ein Elutions pH-Wert eingestellt wird, der oberhalb des Bindungs pH-Wertes liegt und wobei die Menge an nukleinsäurebindender Phase so gewählt ist, dass die Nukleinsäuren in der Probe im Überschuss zu der Bindungskapazität der eingesetzten nukleinsäurebindenden Phase vorliegen.

11. Verwendung nach Anspruch 10, **dadurch gekennzeichnet, dass** die nukleinsäurebindende Phase wenigstens eines der nachfolgenden Merkmale aufweist:

a. die protonierbaren Gruppe(n) weisen einen pKs-Wert von 9, bis 12 vorzugsweise einen pKs-Wert von 10 bis 12 auf; und/oder

b. die protonierbaren Gruppen sind Aminogruppen und liegen nicht in Konjugation mit die Elektronendichte verringernden Gruppen vor; und/oder

c. die nukleinsäurebindende Phase weist funktionelle Gruppen auf, die bei dem Elutions pH-Wert die Freisetzung/Elution der Nukleinsäuren begünstigt, vorzugsweise Kationenaustauscher, insbesondere Carboxylgruppen; und/oder

d. die Bindekapazität der nukleinsäurebindenden Phase liegt in einem Bereich von 1 bis 500$\mu$g Nukleinsäure pro mg nukleinsäurebindender Phase, vorzugsweise 1 bis 200$\mu$g, besonders bevorzugt 10 bis 100$\mu$g Nukleinsäure pro mg nukleinsäurebindender Phase;

e. der Träger weist keine eigenen nukleinsäurebindenden Eigenschaften neben den nukleinsäurebindenden Liganden auf; und/oder

f. der Träger ist ausgewählt aus der Gruppe bestehend aus organischen Polymeren wie Polystyrol und seinen Derivaten, Polyacrylaten und -methacrylaten und ihren Derivaten, Polyurethanen, Nylon, Polyethylen, Polypropylen, Polybutylen und Copolymeren aus diesen Materialien, Polysacchariden und Hydrogelen wie Agarose, Cellulose, Dextran, Sephadex, Sephacryl, Chitosan, anorganischen Trägern, Glas oder weiteren Metall- und Halbmetalloxiden, Boroxid, Trägern mit Metalloberflächen, wie z. B. Gold, magnetischen Partikeln oder Mischungen derselben; und/oder

g. die nukleinsäurebindenden Liganden sind ausgewählt aus der Gruppe bestehend aus primären, sekundären und tertiären Aminen der Formeln

$$R_3N, R_2NH, RNH_2 \text{ und/oder } X\text{-}(CH_2)_n\text{-}Y$$

mit
X = $R_2N$, RNH oder $NH_2$
Y = $R_2N$ oder RNH oder $NH_2$
R = unabhängig voneinander ein verzweigter, unverzweigter oder ringförmiger Alkyl-, Alkenyl-, Alkinyl- oder aromatischer Substituent ist, wobei dieser auch ein oder mehrere Heteroatome enthalten kann;
n= 0 bis 20, vorzugsweise 0 bis 18.

12. Ein Kit zur Isolierung und/oder Aufreinigung einer definierten Menge an Nukleinsäuren aus einer nukleinsäurehaltigen Probe, **dadurch gekennzeichnet, dass** es eine nukleinsäurebindende Phase wie in Anspruch 10 oder 11 definiert aufweist.

13. Das Kit nach Anspruch 12, **dadurch gekennzeichnet, dass** es wenigstens eines der folgenden Merkmale aufweist:

a. einen Bindungspuffer mit einem pH-Wert, der wenigstens eine pH Einheit unterhalb des pKs-Wertes wenigstens einer der protonierbaren Gruppen der nukleinsäurebindenden Phase liegt, und/oder einen Bindungspuffer, der die Einstellung eines solchen pH-Wertes in der Probe ermöglicht; und/oder
b. einen Elutionspuffer mit einem pH-Wert, der eine pH Einheit unterhalb des pKs-Wertes wenigstens einer der protonierbaren Gruppen der nukleinsäurebindenden Phase liegt, und/oder einen Elutionspuffer, der die Einstellung eines solchen pH-Wertes in der Probe ermöglicht.

14. Das Kit nach Anspruch 12 oder 13, **dadurch gekennzeichnet, dass**

a. der Bindungspuffer wenigstens eines der folgenden Merkmale aufweist:

i. einen pH-Wert von 3 bis 8; und/oder
ii. einen pH-Wert von 4 bis 7,5; und/oder
iii. einen pH-Wert von 4,5 bis 7; und/oder
iv. einen pH-Wert von 5,5 bis 7; und/oder
v. einen pH-Wert von 6,5 bis 7; und/oder
vi. eine Salzkonzentration von $\leq 1M$, $\leq 0,5M$, $\leq 0,25M$ oder $\leq 0,1M$; und/oder

b. der Elutionspuffer wenigstens eines der folgenden Merkmale aufweist:

i. einen pH-Wert von 7,5 bis 10; und/oder
ii. einen pH-Wert von 8 bis 9; und/oder
iii. einen pH-Wert von 8,2 bis 8,8; und/oder
iv. eine Salzkonzentration von $\leq 1M$, $\leq 0,5M$, $\leq 0,25M$, $\leq 0,1M$, $\leq 25mM$, $\leq 15mM$ oder $\leq 10mM$; und/oder
v. er ist ausgewählt aus der Gruppe bestehend aus Wasser, biologischem und organischem Puffer.

**Fig. 1**

DNA-Bindung an 2µl Beads

$y = 0{,}5429x + 493{,}94$
$R^2 = 0{,}9967$

Eluat [ng] (y-axis)

DNA in [ng] (x-axis)

**Fig. 2**

Agarosegel

1) 1µl LR-PCR06
2) 10µl Eluat Probe **1**
3) 10µl Eluat Probe **2**
4) 10µl Eluat Probe **3**
5) 10µl Eluat Probe **4**
6) 2µl HML

**Fig. 3:**

| Probe | Konz. Eluat [ng/µl] | Ausbeute Eluat [ng] |
|-------|---------------------|---------------------|
| 1 | 32,23 | 644,6 |
| 2 | 32,03 | 640,6 |
| 3 | 31,44 | 628,8 |
| 4 | 29,03 | 580,6 |

Konz. Eluat [ng/µl]

**Fig. 4**

| PCR | Größe [kb] | Konz. [µg/µl] | Eluat [ng] |
|---|---|---|---|
| LR-PCR1 | 9,9 | 0,027 | 547 |
| LR-PCR2 | 10,1 | 0,027 | 544 |
| LR-PCR3 | 9,2 | 0,027 | 541 |
| LR-PCR4 | 11,0 | 0,026 | 518 |
| LR-PCR5 | 10,0 | 0,027 | 547 |
| LR-PCR6 | 7,6 | 0,028 | 565 |
| LR-PCR7 | 8,9 | 0,028 | 562 |
| LR-PCR8 | 10,1 | 0,027 | 533 |
| LR-PCR9 | 10,1 | 0,026 | 513 |
| LR-PCR10 | 8,7 | 0,027 | 536 |
| LR-PCR11 | 9,6 | 0,025 | 509 |
| LR-PCR12 | 10,7 | 0,024 | 489 |

| Mittelwert [ng] | Std.dev [ng] |
|---|---|
| 533,51 | 22,54 |

| % | Schwankung (%) |
|---|---|
| 100,00 | 4,23 |

Eluat [ng]

**Fig. 5**

Agarosegel der Eluate (Spur 1, 2µl HML; Spuren 2-13, 10µl Eluat LR-PCR1 bis 12; Spur 14, 5µl Lambda HindIII

Europäisches
Patentamt

European
Patent Office

Office européen
des brevets

**EUROPÄISCHER RECHERCHENBERICHT**

Nummer der Anmeldung

EP 09 00 7338

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2006/128776 A1 (QIAGEN GMBH [DE]; HIMMELREICH RALF [DE]; ERBACHER CHRISTOPH [DE]; LOEF) 7. Dezember 2006 (2006-12-07) * Seite 2, Zeile 10 - Seite 3, Zeile 25 * ----- | 1-14 | INV. C12N15/10 |
| Y | WO 99/29703 A2 (DNA RESEARCH INSTR LIMITED [GB]; BAKER MATTHEW JOHN [GB]) 17. Juni 1999 (1999-06-17) * Seite 3, Zeile 5 - Zeile 30 * ----- | 1-14 | |
| Y | WO 02/48164 A2 (DNA RES INNOVATIONS LTD [GB]; BAKER MATTHEW JOHN [GB]) 20. Juni 2002 (2002-06-20) * Anspruch 1 * ----- | 1-14 | |
| Y | WO 00/69872 A2 (PROMEGA CORP [US]) 23. November 2000 (2000-11-23) * Anspruch 1 * ----- | 1-14 | |
| Y | US 2005/130196 A1 (HOFSTADLER STEVEN A [US] ET AL) 16. Juni 2005 (2005-06-16) * Absatz [0022] - Absatz [0030] * ----- | 1-14 | RECHERCHIERTE SACHGEBIETE (IPC) C12N |
| A | Johnson et al.: "'Factory-Proof' long PCR enrichment and amplicon equalization for next generation resequencing applications" 2008, XP002570305 Gefunden im Internet: URL:https://www.invitrogen.com/etc/mediali b/en/filelibrary/Nucleic-Acid-Amplificatio n-Expression-Profiling/PDFs.Par.12017.File .dat/0-076831_SequalPrep%20Application%20N ote%201_0508.pdf> [gefunden am 2010-02-24] * das ganze Dokument * ----- | 1-14 | |

Der vorliegende Recherchenbericht wurde für alle Patentansprüche erstellt

| Recherchenort | Abschlußdatum der Recherche | Prüfer |
|---|---|---|
| München | 25. Februar 2010 | Weikl, Martina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 00 7338

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-02-2010

| Im Recherchenbericht angeführtes Patentdokument | Datum der Veröffentlichung | Mitglied(er) der Patentfamilie | Datum der Veröffentlichung |
|---|---|---|---|
| WO 2006128776    A1 | 07-12-2006 | EP      1893770 A1 | 05-03-2008 |
|  |  | US    2009136926 A1 | 28-05-2009 |
| WO 9929703    A2 | 17-06-1999 | AT       386044 T | 15-03-2008 |
|  |  | AT       218140 T | 15-06-2002 |
|  |  | AU       755342 B2 | 12-12-2002 |
|  |  | AU      1344799 A | 28-06-1999 |
|  |  | BR      9815569 A | 09-10-2001 |
|  |  | CA      2318306 A1 | 17-06-1999 |
|  |  | CN      1281462 A | 24-01-2001 |
|  |  | DE     69805649 D1 | 04-07-2002 |
|  |  | DE     69805649 T2 | 05-12-2002 |
|  |  | DE     69839133 T2 | 05-02-2009 |
|  |  | DK      1036082 T3 | 12-08-2002 |
|  |  | EP      1036082 A2 | 20-09-2000 |
|  |  | ES      2301581 T3 | 01-07-2008 |
|  |  | ES      2177093 T3 | 01-12-2002 |
|  |  | HK      1034520 A1 | 24-03-2006 |
|  |  | JP    2004501054 T | 15-01-2004 |
|  |  | KR   20050088164 A | 01-09-2005 |
|  |  | NO     20002540 A | 07-07-2000 |
|  |  | PT      1036082 E | 31-10-2002 |
| WO 0248164    A2 | 20-06-2002 | AT       283275 T | 15-12-2004 |
|  |  | AU      1620302 A | 24-06-2002 |
|  |  | CA      2432075 A1 | 20-06-2002 |
|  |  | DE     60107468 D1 | 30-12-2004 |
|  |  | DE     60107468 T2 | 29-12-2005 |
|  |  | EP      1345952 A2 | 24-09-2003 |
|  |  | ES      2233560 T3 | 16-06-2005 |
|  |  | JP    2004521881 T | 22-07-2004 |
| WO 0069872    A2 | 23-11-2000 | AT       271605 T | 15-08-2004 |
|  |  | AU       772046 B2 | 08-04-2004 |
|  |  | AU      5126100 A | 05-12-2000 |
|  |  | CA      2372054 A1 | 23-11-2000 |
|  |  | DE     60012318 D1 | 26-08-2004 |
|  |  | DE     60012318 T2 | 04-08-2005 |
|  |  | EP      1179057 A2 | 13-02-2002 |
|  |  | JP      4377550 B2 | 02-12-2009 |
|  |  | JP    2002543979 T | 24-12-2002 |
|  |  | US      6310199 B1 | 30-10-2001 |
|  |  | US    2001014650 A1 | 16-08-2001 |
| US 2005130196    A1 | 16-06-2005 | KEINE |  |

EPO FORM P0461

Für nähere Einzelheiten zu diesem Anhang : siehe Amtsblatt des Europäischen Patentamts, Nr.12/82